# NOUVEAU FASCICULE DE BREVET EUROPEEN

(11) **EP 0 504 055 B2**
(45) Date de publication et mention de la décision concernant l'opposition: **17.03.1999**
(45) Mention de la délivrance du brevet: 04.10.1995
(21) Numéro de dépôt: 92400650.5
(22) Date de dépôt: 12.03.1992
(51) Int. Cl.: A23L 1/09, A23L 1/0522

(54) **Compositions à usage diététique et thérapeutique comprenant des glucides et leurs applications**
Gluciden enthaltend Zusammenstellungen zur Diät und Therapie und ihre Verwendungen
Compositions comprising glucides for use in diet and therapy, and their uses

(30) Priorité: 13.03.1991 FR 9103042
(43) Date de publication de la demande: 16.09.1992
(73) Titulaire: Société des Produits Nestlé S.A., 1800 Vevey (CH)
(72) Inventeur: Audry, Francis, F-14480 Creully (FR); Evard, Daniel, F-94300 Vincennes (FR); Grasset, Etienne, F-92200 Neuilly sur Seine (FR); Jaussan, Véronique, F-14000 Caen (FR)
(74) Mandataire: Kremer, Robert A.M.

(56) Documents cités:
- EP-A- 0 229 938
- EP-A- 0 334 407
- WO-A-89/08988
- FR-A- 2 560 013
- FR-A- 2 622 404
- NEW ENGLAND JOURNAL OF MEDICINE vol. 310, no. 3, Janvier 1984, WALTHAM,MA pages 171 - 175; Y.-T.CHEN: 'cornstarch therapy in type I glycogen-storage disease'
- WORLD PATENTS INDEX LATEST Section Ch, Week 8345, Derwent Publications Ltd.,London, GB; Class D, AN 83-813758
- Compendium "Dieetpreparaten en voedingsmiddelen onder merknaam", 17e Editie 1989/90, De Toorts, Haarlem, The Netherlands, May 1989, p. 102
- Compendium "Dieetpreparaten en voedingsmiddelen onder merknaam", 18e Editie 1990/91, De Toorts, Haarlem, The Netherlands, May 1990, p. 107-108, 112
- Product-Sheet Nutricia "Nieuw Nutrison Vezelverrijkt"
- Nutricia Vademecum 1990, pp. 396-397

## Description

La présente invention concerne de nouvelles compositions destinées à être utilisées en diététique et en thérapeutique et renfermant une combinaison particulière de glucides et leurs applications.

De nombreux produits à base de glucides utilisés en diététique ou en thérapeutique et plus particulièrement en nutrition thérapeutique existent déjà sur le marché. Ces produits possèdent une fraction glucidique contenant, en proportion variable, des polysaccharides polymères de glucose de poids moléculaire variable sous forme de dextrine maltose, de disaccharides (maltose, saccharose, lactose), de mono-saccharide (glucose). L'ensemble de ces glucides sont rapidement assimilables.

L'intérêt de l'ingestion de sucres à absorption lente pour éviter les hypoglycémies nocturnes a déjà été démontré en cas de glycogénose de type 1 par Chen Y. T., Cornblath M., Sidbury J. : Cornstarch therapy in type 1 glycogen-storage disease. New England Journal of Medicine 1984 ; 310 : 171-175.

Ces auteurs utilisaient cependant de l'amidon de maïs natif, cru et mis en suspension extemporanément. Un traitement par chauffage était impossible car le caractère de sucre lent était alors perdu.

La demande de brevet international WO 89/08988 décrit des substituts de produit laitier contenant de la maltodextrine et une huile dans une matrice d'hydrocarbures ou de protéines destinés à reproduire les sensations gustatives produites en bouche par les graisses et les crèmes lactiques. L'exemple 9 de cette demande de brevet décrit une poudre composée notamment de maltodextrine et d'une huile végétale dans une matrice d'amidon de maïs qui diluée dans de l'eau à température de 100°C produit une crème épaissie exempte de cholestérol et d'acides gras saturés.

La demande de brevet européen EP 0 229 938 décrit une composition nutritionnelle pour l'obtention de produits crémeux épaissis contenant des lipides, de l'amidon hydrolysé, de l'amidon comme épaississant et un émulsionnant. Ces compositions se présentent sous forme de poudre ou d'agglomérat qui sont ensuite diluées ou dispersées dans de l'eau à une température de 100°C pour préparer un produit alimentaire final épaissi de type crème, pudding, velouté, etc ...

Le problème que se propose de résoudre la présente invention est d'obtenir des compositions nutritionnelles liquides, tout en comportant une fraction glucidique à absorption lente représentant une part importante du poids total des compositions.

A la connaissance de la demanderesse, ce type de composition n'existe pas dans l'art antérieur. En effet, il est connu que les glucides à absorption lente tels que les amidons modifiés ou la pectine donnent naissance, après cuisson en solutions aqueuses, à des gels stables ce qui augmente la viscosité des compositions les contenant et ne permet pas d'obtenir des compositions liquides qui sont plus aptes à l'utilisation nutritionnelle des produits par exemple par sonde digestive.

La présente invention a donc pour objet des compositions nutritionnelles liquides caractérisées en ce que la fraction glucidique qu'elles contiennent, renferme au moins les deux types de glucides suivants :
- des polymères de glucose,
- des glucides d'absorption lente.

La présente invention a notamment pour objet des compositions nutritionnelles liquides comprenant une fraction glucidique, une fraction lipidique et une fraction protéique caractérisées en ce que la fraction glucidique renferme au moins les deux types de glucides suivants :
- des polymères de glucose,
- des glucides d'absorption lente.

Par polymères de glucose, on entend principalement les dextrines maltoses.

Par glucides à absorption lente, on entend les produits ayant un index glycémique significativement plus bas que le glucose et que les indices glycémiques publiés pour les constituants habituels : dextrine maltose, disaccharides tels que le maltose, le saccharose. Les glucides à absorption lente sont des glucides qui entraînent une réponse glycémique plus lente et plus faible que les glucides dits rapides. Cette réponse plus lente et plus faible résulte d'une absorption digestive plus lente.

Les glucides à absorption lente peuvent être par exemple des amidons modifiés ou des fibres solubles.

Les amidons modifiés sont actuellement largement utilisés dans l'industrie agro alimentaire et pharmaceutique, ceci en raison de leurs propriétés fonctionnelles : ils permettent d'obtenir après cuisson en suspension aqueuse des gels stables. Les propriétés de gel et la viscosité sont augmentées de la sorte. Ce qui est un avantage en général est, au contraire, pour les compositions liquides de l'invention un inconvénient.

Les amidons modifiés pourraient être des sucres à absorption lente, ce qui est recherché pour l'invention, mais le fait de favoriser la formation de gels visqueux en solution aqueuse n'est pas compatible avec l'utilisation nutritionnelle finale du produit. Contrairement à l'enseignement de l'art antérieur, nous avons essayé de déterminer si néanmoins certains amidons et procédés de fabrication pourraient permettre la réalisation de l'invention.

Un certain nombre d'amidons modifiés ont été préalablement sélectionnés pour répondre aux contraintes technologiques, à savoir, rentrer dans une préparation prête à l'emploi, liquide et stérilisable de manière industrielle.

Pour cela quatre critères fonctionnels sont indispensables :
- la résistance au traitement thermique de type U.H.T. c'est-à-dire conserver une fluidité à chaud,
- une viscosité stable aux variations de pH et de température,
- une bonne solubilité dans l'eau,
- une résistance à la rétrogradation et au cisaillement.

Trois amidons modifiés après ces études de propriétés fonctionnelles ont été retenus, le Cleargum CB 90 (Roquette Frères S.A., Lestrem, France), Snowflake 6090 (Cerestar S.A. Haubourdin, France) et l'Encapsol 855 (National Starch et Chemical S.A., Villefranche, France).

Parmi les fibres solubles on peut citer par exemple, la pectine ou certaines fibres de soja.

Comme indiqué précédemment, la pectine a normalement également une action gélifiante dans une solution complexe lors des traitements de stérilisation par chauffage et augmente la viscosité des produits terminés prêts à l'emploi et de ce fait gêne l'écoulement de produits de nutrition par sonde digestive. C'est pour cette raison qu'elle n'est actuellement pas employée dans les produits de nutrition artificielle stérilisables, susceptibles d'être administrés par sonde, bien que l'intérêt des fibres solubles, notamment de la pectine, ait été souligné par certains auteurs. La pectine est, en effet, lentement métabolisée par le côlon et réalise un apport d'énergie étalé sur 24 heures (Pomare E. W., Branch W. J., Cummings J. H. : Carbohydrate fermentation in the human colon and its relation to acetate concentrations in venous blood. Journal of Clinical Investigation 1985 ; 75 : 1448-1454). L'ingestion de fibres solubles est également recommandée chez les patients atteints de diabète sucré.

La présente invention a consisté à rechercher et tester différentes fibres et pectines de façon à répondre d'une part aux contraintes d'un produit liquide, prêt à l'emploi et stérilisable et d'autre part contribuer à un effet nutritionnel.

Nous avons étudié l'influence de différentes pectines et fibres sur le comportement rhéologique du produit liquide à stériliser. Par des tests physiques et physico-chimiques nous avons sélectionné des fibres et pectines répondant aux critères suivants :
- avoir une faible sensibilité aux ions Calcium,
- ne pas former de gels dans un milieu peu ou moyennement sucré,
- ne pas donner de gel très ferme aux pH compris entre 6,0 et 7,5 lorsque la solution renferme au moins 60 % de sucres,
- être compatible avec des polymères de glucose tels que des amidons modifiés après un traitement de stérilisation par un chauffage.

C'est donc également une caractéristique très importante et inattendue de la présente invention de pouvoir comporter des amidons et fibres solubles telle que la pectine et néanmoins subir une stérilisation par chauffage et conserver une viscosité inférieure à 0,05 kg x m⁻¹ x sec⁻¹ (50 centipoises).

La présente invention a particulièrement pour objet, les compositions telles que décrites précédemment caractérisées en ce qu'elles sont sous une forme stérilisée stable prête à l'emploi.

Contrairement aux compositions de l'art antérieur décrites notamment par Chen. et coll., la présente invention permet d'utiliser des amidons modifiés comme le Cleargum CB 90, stérilisables sans modification physique et donc de préparer des compositions liquides prêtes à l'emploi. Le degré de polymérisation du Cleargum CB 90 a été mesuré avant et après stérilisation par UHT (7 secondes à 150 degrés Celsius). Les résultats, obtenus par HPLC sur colonne HPIC-AS6, ont été les suivants : absence de mono- et de disaccharides et non détection de polymères de glucose contenant moins de 11 molécules de cet élément.

La présente invention a plus particulièrement pour objet les compositions telles que décrites précédemment, caractérisées en ce que la fraction glucidique représente 5 à 40 % du poids total.

Le caractère liquide des compositions, objet de la présente invention est très important, l'invention a donc particulièrement pour objet, les compositions telles que décrites précédemment, caractérisées en ce que leur viscosité est inférieure à 0,05 kg x m⁻¹ x sec⁻¹ (50 centipoises), de préférence inférieure à 0,03 kg x m⁻¹ x sec⁻¹ (30 centipoises).

On préfère de plus les compositions ayant conservé une viscosité inférieure à 0,02 kg x m⁻¹ x sec⁻¹ -(20 centipoises) (mesure par appareil Contraves Rheomat 108 à une température ambiante de 20 degrés Celsius) et permettant éventuellement une administration par sonde digestive.

Parmi les compositions de l'invention, on préfère les compositions dans lesquelles la fraction glucidique renferme au moins les glucides suivants :
- maltodextrines,
- amidon modifié,
- fibres solubles.

L'invention a plus particulièrement pour objet, les compositions caractérisées en ce que le composant de la fraction glucidique ayant un index glycémique mesuré chez l'homme sain, inférieur ou égal à 70 représente au moins 20 % du poids de glucides totaux présents dans la composition.

L'index glycémique est déterminé selon la méthode décrite dans la publication suivante, Wolever T.M.S., Jenkins D.J.A. : The use of the Glycemic index in predicting the blood glucose response to mixed meals. American Journal of Clinical Nutrition 1986 ; 43 : 167-172. L'élévation de la glycémie intégrée sur les deux heures qui suivent l'ingestion du glucide étudié est comparée à celle obtenue après ingestion de la même quantité de glucose (base 100).

De telles compositions peuvent être réalisées par l'incorporation d'amidons modifiés comme le Cleargum CB 90, cet ingrédient étant fourni à titre d'exemple et non limitatif. Des fibres solubles (par exemple pectine) peuvent également être utilisées.

De préférence, dans les compositions selon la présente invention, les glucides simples ou complexes, dérivés de l'amidon ou non, représentent au moins 50 % de l'apport énergétique (la base de calcul étant de 16,744 kilojoules/g (4 kcal/g) de glucides présents dans la composition).

Les compositions selon la présente invention, peuvent ne pas comporter d'addition de saccharose. L'obtention d'un goût sucré s'obtient donc dans ce cas par l'utilisation d'un autre produit sucrant : monosaccharide tel que le fructose, édulcorant intense tel que l'aspartam ou l'acésulfam K.

Eien entendu, les compositions de l'invention peuvent apporter tous les éléments nutritifs nécessaires, autres que glucidiques, en proportions étudiées. Elles peuvent renfermer ainsi des protéines assurant 10 à 17 % de l'A.E.T. (apport énergétique total), des vitamines (A, D, E, C, B1, B2, PP, B6, B12, acide folique, biotine, B5, K1, choline).

Les compositions selon l'invention peuvent aussi assurer une couverture des besoins physiologiques en éléments minéraux.

Elles peuvent également renfermer une fraction lipidique assurant 20 à 40 % de l'A.E.T..

Cette fraction lipidique peut avoir approximativement la composition suivante (pour 2,670 g de lipides) :

| | |
|---|---|
| Huile de colza LEAR | 1,281 g |
| Triglycérides à chaîne moyenne (TCM) | 0,670 g |
| Huile de maïs | 0,400 g |
| Lécithine de soja | 0,188 g |
| Stéarate de glycérol | 0,131 g |

A titre indicatif, si ces 2,670 g de lipides sont incorporés à l'invention de manière à apporter 30 % de l'A.E.T., la fraction lipidique apportera les acides gras essentiels suivants :

| | |
|---|---|
| Acide linoléique | 6,4 % de l'A.E.T. |
| Acide alpha-linolénique | 1,6 % de l'A.E.T. |

Cette composition lipidique permet d'apporter une quantité suffisante d'acides gras essentiels (8 % de l'A.E.T.) et de conserver un rapport acide alpha-linolénique/acide linoléique supérieur à 1/10.

Les TCM de l'invention peuvent être remplacés par les éléments suivants :
- huile d'olive ou huile de tournesol riche en acide oléique afin d'augmenter la proportion d'acides gras mono-insaturés,
- huile d'onagre riche en acide gamma-linolénique,
- triglycérides de synthèse contenant en position 1, 2 ou 3 des acides gras poly-insaturés tels que acide gamma-linolénique (C18 : 3,n-6), acide dihomogamma-linolénique (C20 : 3,n-6), acide eicosapentaénoïque (C20 : 5,n-3). La fraction lipidique peut en particulier contenir les triglycérides de synthèse correspondant au brevet déposé en France délivré sous le numéro 2,515,174. Elle pourrait en particulier, à titre d'exemple, contenir le 1-3 dioctanoyl eicosapentaénoyl glycérol. En effet, certaines données récentes suggèrent que l'ingestion d'acide eicosapentaénoïque pourrait avoir un effet bénéfique dans la thérapeutique des patients présentant un diabète insulinodépendant (Jensen T., Stender S., Goldstein K., Holmer G., Deckert T. : Partial normalization by dietary cod-liver oil of increased microvascular albumin leakage in patients with insulin dependent diabetes and albuminuria. New England Journal of Medicine 1989 ; 321 : 1572-1577),
- huile de colza LEAR pour augmenter l'apport en acide alphalinolénique,
- huile de maïs ou de soja pour augmenter l'apport en acide linoléique.

La présente invention a notamment pour objet des préparations liquides, prêtes à l'emploi destinées à l'alimentation par voie orale ou à la nutrition par voie digestive de patients, adultes ou enfants atteints d'anomalies du métabolisme glucidique les rendant susceptibles de présenter des variations excessives de la glycémie (hypo ou hyperglycémie), en particulier diabète sucré et intolérance au glucose. En outre, les affections caractérisées par l'impossibilité de supporter un jeûne prolongé du fait d'un risque accru d'hypoglycémies pourraient bénéficier de,l'invention : glycogénoses, hypoglycémies de l'enfant par manque de substrat (hypoglycémies récurrentes avec cétose).

Les compositions de l'invention sont utilisables en diététique, réanimation et thérapeutique.

La demande a ainsi pour objet l'application des compositions telles que définies précédemment, pour la préparation d'aliments ou de suppléments alimentaires répondant à des besoins spécifiques nutritionnels.

Les compositions de l'invention sont utilisables lorsqu'une assistance nutritionnelle est nécessaire, soit parce que l'ingestion d'une alimentation normale est devenue impossible ou insuffisante du fait de l'état du malade, soit en supplémentation de l'alimentation normale, par exemple en collation au milieu de la matinée et au coucher, ces exemples n'étant pas limitatifs. Les compositions de l'invention ont un intérêt particulier en cas d'anomalies du métabolisme glucidique exposant à un risque de variation excessif de la glycémie (hyper- ou hypoglycémie) telles que le diabète sucré insulinodépendant ou non, intolérance au glucose, maladie avec risque d'hypoglycémie prévisible (hypoglycémie récurrente avec cétose, hypoglycémie par manque de substrat, anomalies congénitales du métabolisme des glucides ou des lipides exposant à un risque accru d'hypoglycémie en cas de jeûne).

Elle peut également être utilisée en remplacement des repas dans le cadre d'un régime hypocalorique chez un patient obèse présentant un diabète ou une intolérance au glucose.

La présente invention a également pour objet les aliments ou suppléments alimentaires comprenant une composition telle que définie ci-dessus, en association avec un véhicule neutre convenant à l'administration orale ou entérale.

La présente invention a également pour objet, à titre de médicaments, les compositions telles que définies ci-dessus, ainsi que les compositions pharmaceutiques comprenant comme principe actif, un médicament tel que défini ci-dessus, en association avec un véhicule neutre convenant à l'administration orale ou entérale.

La présente invention a également pour objet une méthode de traitement diététique caractérisé en ce que l'on administre les compositions telles que définies ci-dessus, ou les aliments ou suppléments alimentaires tels que décrits ci-dessus.

Par ailleurs, l'invention a également pour objet un procédé de préparation des compositions telles que définies ci-dessus, caractérisé en ce que l'on prépare à partir d'une phase lipidique, d'abord une émulsion en phase aqueuse comprenant certains glucides d'absorption lente, de préférence des fibres solubles et ajoute à cette émulsion une phase hydrosoluble composée d'une fraction protéique et d'une fraction glucidique comprenant des polymères de glucose et au moins 20 % en poids de glucides d'absorption lente.

Plus précisément, l'invention a pour objet le procédé tel que défini ci-dessus, caractérisé en ce que l'émulsion en phase aqueuse comprend 0,5 % de fibres solubles, de préférence la pectine.

Enfin l'invention a précisément pour objet un procédé de préparation des compositions telles que décrites ci-dessus, caractérisé en ce que le mélange entre l'émulsion et la phase hydrosoluble est ensuite stérilisé puis homogénéisé.

Les exemples suivants illustrent l'invention sans toutefois la limiter.

### EXEMPLE 1 :

On a préparé la préparation diététique et thérapeutique liquide à usage oral ou entéral, pour administration par sonde digestive, de formule suivante :

### Composition centésimale :

- glucides 11,000 g

| | | |
|---|---|---|
| dont : | Maltodextrines | 6,925 g |
| | Cleargum CB 90 | 3,200 g |
| | Fibres/pectines | 0,500 g |

| | Lactose apporté par les ingrédients | |
|---|---|---|
| | ingrédient de la fraction protéique | 0,375 g |
| | (Acésulfam K | 15,000 mg) |

- lipides 2,670 g

| | | |
|---|---|---|
| dont : | TCM | 0,670 g |
| | Huile de maïs | 0,400 g |
| | Huile de colza LEAR | 1,281 g |
| | Lécithine de soja | 0,188 g |
| | Stéarate de glycérol | 0,131 g |

- protéine 3,000 g

| | | |
|---|---|---|
| dont : | Protéines de lactosérum 75 % | 2,750 g |
| | Lait sec écrémé | 0,249 g |
| | Carnitine | 2,500 mg |

- vitamines et minéraux selon la dixième édition (1989) des RDA (Subcommittee on the Tenth Edition of the RDAs, Food and Nutrition Board, Commission on Life Sciences, National Research Council : Recommended Dietary Allowances, 10 th edition. National Academy Press, Washington DC : 1989).
- eau QSP 100 ml.

### Technique de fabrication.

La préparation de 2 500 litres de l'exemple 1 est réalisé selon un procédé en 3 étapes, comme suit :

Le matériel utilisé est constitué de :
- un mélangeur de 400 litres,
- une cuve en acier inoxydable de 4 000 litres munie :
   . d'un système d'agitation
   . d'un système de chauffage et de refroidissement,
- un échangeur à plaques doté de trois circuits :
   . un refroidissement à 5°C
   . un refroidissement à 15°C
   . un chauffage,
- de 2 pompes centrifuges,
- un broyeur.

### Constituants

| | | |
|---|---|---|
| 1 - | Pectine | 13,77 kg |
| | Carraghénates | 500,00 g |
| | Phosphate disodique | 500,00 g |
| | Lécithine de soja | 4,70 kg |
| | Huile de maïs | 10,00 kg |
| | Huile de colza LEAR | 32,03 kg |
| | T.C.M. | 16,75 kg |
| | Stéarate de glycérol | 3,27 kg |
| | Eau osmosée | 250,00 litres |
| 2 - | Protéines de lait | 68,73 kg |
| | Eau osmosée | 900,00 litres |
| 3 - | Maltodextrines | 180,30 kg |
| | Lait sec écrémé | 17,50 kg |
| | Amidon modifié | 89,30 kg |
| | Acésulfam K | 375,00 g |
| | Eau osmosée | 250,00 litres |
| Eau osmosée qsp | | 2 500,00 litres |

### Le procédé de fabrication est le suivant :

1 est dispersé dans le mélangeur à 60°C, puis mélangé avec 2. 1 et 2 sont maintenus sous agitation pendant 2 minutes et le mélange est vidé dans la cuve de 4 000 litres en passant par le broyeur.

On abaisse la température à 35°C, grâce à la section de refroidissement de l'échangeur, puis on incorpore successivement dans le mélangeur, 3 à une température inférieure à 40°C, en travaillant en circuit fermé (mélangeur-cuve-échangeur-mélangeur). Le mélange 1, 2 et 3 est recirculé en circuit fermé pendant 5 minutes puis vidé complètement dans la cuve de 4 000 litres.

Une quantité d'eau osmosée est introduite dans le circuit de façon à ajuster le volume de la cuve à 2 450 litres. Une mesure de densité du produit de la cuve et l'ajustement en poids par pesons sont effectués.

Le pH est ajusté à 7,10 par addition d'une solution d'hydroxyde de Potassium à 2N.

Le mélange ainsi obtenu est alors dégazé, stérilisé UHT et homogénéisé à une pression d'environ 200 kg/cm² à 75°C et enfin réparti aseptiquement dans des boîtes métalliques serties. Le produit est alors stocké à la température ambiante.
Stabilité : aucune remontée de matière grasse, ni crémage, ni gélification du produit n'est observé après 90 heures.

### EXEMPLE 2 :

Une solution diététique et thérapeutique à usage oral ou entéral est préparée selon l'exemple 1, excepté que d'une part la pectine est substituée par des fibres de soja et l'acésulfam K remplacé par du fructose.

### Composition centésimale :

| | | |
|---|---|---|
| - protéines : protéines de lait (N x 6,25) | | 3,00 g |
| - lipides : | | 2,67 g |
| dont : | TCM | 0,67 g |
| | Huile de maïs | 0,40 g |
| | Huile de colza LEAR | 1,28 g |
| | Lécithine de soja | 0,19 g |
| | Stéarate de glycérol | 0,13 g |

### Etude clinique du produit de l'exemple 1 :

Une boîte de 375 ml du produit de l'invention a été consommée quotidiennement par six diabétiques de type II, deux hommes et quatre femmes âgés de 42 ans à 74 ans (médiane : 60,5 ans), avec surcharge pondérale de 26,7 % à 49,5 % (médiane : 38,8 %), pendant une durée de 21 jours consécutifs d'hospitalisation pour 4 d'entre eux et une durée de 28 jours consécutifs en ambulatoire pour les 2 autres.

Chaque boîte du produit de l'invention apportait 300 Kcal, soit comme susbtitut du petit déjeûner chez les 4 patients hospitalisés, soit comme substitut partiel du déjeûner de midi chez les patients ambulatoires. Les 6 patients étaient soumis à un régime hypocalorique personnalisé apportant journalièrement 4186 à 5860,4 kilojoules (1000 à 1400 kcal) (médiane : 5023,2 kilojoules (1200 kcal)) dont 1255,8 kilojoules (300 kcal) du produit de l'invention. Aucun des 6 patients n'a présenté au cours de cet essai clinique prolongé de signe clinique d'intolérance digestive (pas de nausée, pas de vomissement, pas de diarrhée, pas d'anorexie), ou extradigestive.

L'adhérence à la prise du produit de l'invention a été excellente : 375 ml du produit de l'invention 1255,8 kilojoules (300 kcal) ont été effectivement consommés quotidiennement par chacun des 6 patients, pendant 21 jours pour 4 d'entre eux et pendant 28 jours pour les 2 autres. Chez aucun des 6 patients n'a été notée l'apparition d'anomalies de la formule sanguine, du bilan hépatique (ASAT, ALAT, Phosphatases alcalines), de la fonction rénale (créatininémie), de l'équilibre électrolytique plasmatique (natrémie, kaliémie, réserve alcaline).

La perte de poids de 0,4 à 5,2 kg , notée chez les 6 patients (médiane : 2,7 kg), s'est accompagnée d'une baisse chez 5 patients sur 6 de la glycémie basale de 0,5 à 6 mmol/l (médiane : 3,8 mmol/l) et postprandiale de 1,4 à 8,3 mmol/l (médiane : 3,4 mmol/l).

Au total, 375 ml du produit de l'invention pris quotidiennement pendant 3 à 4 semaines par 6 diabétiques de type II ont été parfaitement tolérés tant sur le plan clinique que biologique.

## Revendications

1. Composition nutritionnelle liquide comprenant une fraction glucidique, une fraction lipidique, et une fraction protéique, caractérisée en ce que la fraction glucidique renferme au moins les deux types de glucides suivants :
- des polymères de glucose
- des glucides d'absorption lente, choisis parmi les amidons modifiés et les fibres solubles,
le composant de ladite fraction glucidique ayant un index glycémique mesuré chez l'homme sein, inférieur ou égal à 70 représentant au moins 20% en poids des glucides totaux présents dans la composition,
ladite composition nutritionnelle restant liquide après stérilisation par traitement thermique.

2. Composition selon la revendication 1, caractérisée en ce qu'elle est sous une forme stérilisée stable prête à l'emploi.

3. Composition selon la revendication 1 ou 2, caractérisée en ce que la fraction glucidique représente 5 à 40 % du poids total.

4. Composition selon l'une quelconque des revendications 1 à 3, caractérisée en ce que sa viscosité est inférieure à 0,05 kg x m⁻¹ x sec ^{- 1} (50 centipoises), de préférence inférieure à 0,03 kg x m ⁻¹ x sec ^{- 1} (30 centipoises).

5. Composition selon l'une quelconque des revendications 1 à 4, caractérisée en ce que la fraction glucidique renferme au moins les glucides suivants :
- maltodextrines,
- amidon modifié
- fibres solubles.

6. Composition selon la revendication 5, caractérisée en ce que dans la fraction glucidique les fibres solubles sont des pectines.

7. Composition selon l'une quelconque des revendications 1 à 6, caractérisée en ce qu'elle est préparée
- par émulsion d'une phase lipidique dans une phase aqueuse comprenant certains glucides d'absorption lente, de préférence des fibres solubles, et
- par adjonction à cette émulsion d'une phase hydrosoluble composée d'un fraction protéique et d'une fraction glucidique comprenant les polymères de glucose et au moins 20% en poids des glucides d'absorption lente.

8. Composition selon la revendication 7, caractérisée en ce que l'émulsion en phase aqueuse comprend 0,5% de fibres solubles, de préférence de la pectine.

9. Composition selon la revendication 7 ou 8, caractérisée en ce que le mélange entre l'émulsion et la phase hydrosoluble est ultérieurement stérilisé puis homogénéisé.

10. Utilisation des compositions selon l'une quelconque des revendications 1 à 9, pour la préparation d'aliments ou de suppléments alimentaires destinés à prévenir ou traiter les variations excessives de la glycémie.

11. Procédé de préparation des compositions selon la revendication 1, caractérisé en ce que l'on prépare à partir d'une phase lipidique, d'abord une émulsion en phase aqueuse comprenant certains glucides d'absorption lente, de préférence des fibres solubles, et on ajoute à cette émulsion une phase hydrosoluble composée d'une fraction protéique et d'une fraction glucidique comprenant des polymères de glucose et au moins 20% en poids de glucides d'absorption lente.

12. Procédé selon la revendication 11, caractérisé en ce que l'émulsion en phase aqueuse comprend 0.5% de libres solubles, de préférence la pectine.

13. Procédé selon la revendication 11 ou 12, caractérisé en ce que le mélange entre l'émulsion et la phase hydrosoluble est ensuite stérilisé puis homogénéisé.

## Claims

1. Liquid nutritional composition comprising a glucide component, a lipid component and a protein component, characterized in that the glucide component comprises at least the following two glucide types:
- glucose polymers,
- slow absorption glucides selected from modified starches and soluble fibres,
the fraction of said glucide component having a glycaemic index measured in a healthy human lower than or equal to 70 being at least 20% by weight of the total glucides present in the composition,
said nutritional composition remaining liquid after heat sterilization.

2. Composition according to Claim 1, characterized in that it is in a ready-to-use stable sterilized form.

3. Composition according to Claim 1 or 2, characterized in that the glucide component is present in an amount of 5 to 40% of the total weight.

4. Composition according to any one of Claims 1 to 3, characterized in that its viscosity is below 0.05 kg × m⁻¹ × sec⁻¹ (50 centipoises), preferably below 0.03 kg × m⁻¹ × sec⁻¹ (30 centipoises).

5. Composition according to any one of Claims 1 to 4, characterized in that the glucide component comprises at least the following glucides:
- maltodextrins,
- modified starch,
- soluble fibres.

6. Composition according to Claim 5, characterized in that, in the glucide component, the soluble fibres are pectins.

7. Composition according to any one of Claims 1 to 6, characterized in that it is prepared
- by emulsification of a lipid phase in an aqueous phase comprising some slow absorption glucides, preferably soluble fibres, and
- by addition to this emulsion of a water-soluble phase composed of a protein component and of a glucide component comprising glucose polymers and at least 20% by weight of slow absorption glucides.

8. Composition according to Claim 7, characterized in that the emulsion comprises 0.5% of soluble fibres, preferably pectin, in the aqueous phase.

9. Composition according to Claim 7 or 8, characterized in that the mixture of the emulsion and the water-soluble phase is next sterilized and then homogenized.

10. Use of the compositions according to any one of Claims 1 to 9 for the preparation of foodstuffs or food supplements intended to prevent or treat excessive variations in glycaemia.

11. Process for the preparation of compositions according to Claim 1, characterized in that first an emulsion in an aqueous phase comprising some slow absorption glucides, preferably soluble fibres, is prepared from a lipid phase, followed by adding to this emulsion a water-soluble phase composed of a protein component and of a glucide component comprising glucose polymers and at least 20% by weight of slow absorption glucides.

12. Process according to Claim 11, characterized in that the emulsion comprises 0.5% of soluble fibres, preferably pectin, in the aqueous phase.

13. Process according to Claim 11 or 12, characterized in that the mixture of the emulsion and the water-soluble phase is next sterilized and then homogenized.

## Patentansprüche

1. Flüssige Ernährungszusammensetzung enthaltend eine Kohlehydratfraktion, eine Lipidfraktion und eine Proteinfraktion, dadurch gekennzeichnet, daß die Kohlehydratfraktion wenigstens die folgenden zwei Kohlehydratarten enthält:
- Glucosepolymere,
- Kohlehydrate langsamer Absorption, ausgewählt aus der Gruppe der modifizierten Stärken und löslichen Fasern,
wobei der Bestandteil der Kohlehydratfraktion mit einem am gesunden Menschen gemessenen Glykämieindex von kleiner oder gleich 70 wenigstens 20 Gew.-% der in der Zusammensetzung vorhandenen Gesamtkohlehydrate ausmacht,
welche Ernährungszusammensetzung nach der Sterilisation durch Wärmebehandlung flüssig bleibt.

2. Zusammensetzung nach Anspruch 1, dadurch gekennzeichnet, daß sie in einer stabilen, anwendungsfertigen sterilisierten Form vorliegt.

3. Zusammensetzung nach Anspruch 1 oder 2, dadurch gekennzeichnet, daß die Kohlehydratfraktion 5 bis 40 % des Gesamtgewichts ausmacht.

4. Zusammensetzung nach einem der Ansprüche 1 bis 3, dadurch gekennzeichnet, daß ihre Viskosität weniger als 0,05 kg x m⁻¹ x sec⁻¹ (50 Centipoise), vorzugsweise weniger als 0,03 kg x m⁻¹ x sec⁻¹ (30 Centipoise) beträgt.

5. Zusammensetzung nach einem der Ansprüche 1 bis 4, dadurch gekennzeichnet, daß die Kohlehydratfraktion mindestens die folgenden Kohlehydrate umfaßt:
- Maltodextrine,
- modifizierte Stärke,
- lösliche Fasern.

6. Zusammensetzung nach Anspruch 5, dadurch gekennzeichnet, daß die in der Kohlehydratfraktion vorhandenen löslichen Fasern Pecktine sind.

7. Zusammensetzung nach einem der Ansprüche 1 bis 6, dadurch gekennzeichnet, daß sie hergestellt worden ist
- durch Emulsion einer Lipidphase in einer wäßrigen Phase, die bestimmte Kohlehydrate langsamer Absorption, vorzugsweise lösliche Fasern, enthält und
- durch Zugabe einer wasserlöslichen Phase, die aus einer Proteinfraktion und einer Kohlehydratfraktion, enthaltend Glucosepolymere und wenigstens 20 Gew.-% Kohlehydrate langsamer Absorption, zusammengesetzt ist.

8. Zusammensetzung nach Anspruch 7, dadurch gekennzeichnet, daß die Emulsion in wäßriger Phase 0,5 % lösliche Fasern, vorzugsweise Pektin, enthält.

9. Zusammensetzung nach Anspruch 7 oder 8, dadurch gekennzeichnet, daß die Mischung aus der Emulsion und der wasserlöslichen Phase schließlich sterilisiert und dann homogenisiert wird.

10. Verwendung der Zusammensetzungen nach einem der Ansprüche 1 bis 9 für die Herstellung von Nahrungsmitteln oder Nahrungsmittelzusätzen, die zur Vorbeugung oder zur Behandlung von übermäßigen Schwankungen der Glykämie bestimmt sind.

11. Verfahren zur Herstellung der Zusammensetzungen nach Anspruch 1, dadurch gekennzeichnet, daß man ausgehend von einer Lipidphase zunächst eine Emulsion in wäßriger Phase bildet, die bestimmte Kohlehydrate langsamer Absorption, vorzugsweise lösliche Fasern, enthält, und zu dieser Emulsion eine wasserlösliche Phase, die aus einer Proteinfraktion und einer Kohlehydratfraktion, die Glucosepolymere und mindestens 20 Gew.-% Kohlehydrate langsamer Absorption enthält, zugibt.

12. Verfahren nach Anspruch 11, dadurch gekennzeichnet, daß die Emulsion in wäßriger Phase 0,5 % lösliche Fasern, vorzugsweise Pektin, enthält.

13. Verfahren nach Anspruch 11 oder 12, dadurch gekennzeichnet, daß die Mischung aus der Emulsion und der wasserlöslichen Phase anschließend sterilisiert und dann homogenisiert wird.
